# EUROPEAN PATENT APPLICATION

(11) **EP 2 641 584 A1**
(43) Date of publication of application: **25.09.2013**
(21) Application number: 12160209.8
(22) Date of filing: 19.03.2012
(51) Int. Cl.: A61K 8/34, A61K 8/67, A61K 8/92, A61K 8/97, A61Q 19/00, A61K 36/738, A61P 17/04

(54) **Cosmetic skin composition with soothing effect and its use**

(71) Applicant: Coty Germany GmbH, 55116 Mainz (DE)
(72) Inventor: Doucet, Olivier, 06190 Roquebrune Cap Martin (FR); Pujos, Muriel, F-06200 Nice (FR); Robert, Cécile, F-06300 Nice (FR); Desriaux, Elisabeth, F-06500 Menton (FR); Bernini, Dorothee, MC-98000 Monaco (MC)
(74) Representative: Gulde Hengelhaupt Ziebig & Schneider

(57) **Abstract**

The present invention relates to a composition with excellent soothing activity and significant and long lasting hydrating effect on skin and its use for soothing the skin. The composition comprises a hydrophilic complex containing a Rosa centifolia flower extract with significant soothing activity, although the Rosa centifolia flower extract is contained in the composition in very small amounts. The composition may be formulated and used as lotion, cream or emulsion.

## Description

The present invention relates to a composition with excellent soothing activity and significant and long lasting hydrating effect on skin and its use for soothing the skin by applying it on skin areas irritated by environmental conditions. The composition comprises a hydrophilic complex containing a Rosa centifolia flower extract with significant soothing activity, although the Rosa centifolia flower extract is contained in the composition in very small amounts. The composition may be formulated and used as lotion, cream or emulsion.

According to the invention irritated skin means skin which is irritated by environmental conditions, e.g. pollution, UV radiation or air conditioning in closed rooms. The features of irritated skin which is caused by these environmental conditions are redness, itching, tightening and/or swelling. Sometimes irritated skin shows light inflammatory reactions.

It is well known in cosmetics to apply to irritated skin vegetal extracts like centella asiatica, avena sativa (oat) extract or chamomile extract, but their use may induce skin allergy reactions. For instance, the use of chamomile-containing preparations is contraindicated in persons with hypersensitivity to ragweed pollens. Due to its content in natural bisabolol, it may induce contact dermatitis, whereas synthetic bisabolol does not. Several cases of oat sensitization have been reported in children with atopic dermatitis with cosmetics containing oat proteins and it is suggested that they should avoid the use of such products. Centella asiatica is another sensitizer which can induce allergic contact dermatitis.

It was the problem of the present invention to provide a composition with superior soothing and moisturizing effects on skin which does not have the disadvantages of the prior art.

It was found by the inventors of the present application that this problem can be solved with a cosmetic composition which comprises 0,1 to 3,0 wt%, preferably 0,3 to 2,0 wt%, of a hydrophilic complex containing 1,5 to 3,0 wt% of Rosa centifolia flower extract which was obtained by cryoextraction of Rosa centifolia flowers; 0,1 to 10,0 wt%, preferably 1,0 to 5,0 wt%, of Rosa centifolia flower water, 0,1 to 1,0 wt% anti-irritants and water and cosmetically acceptable auxiliary substances up to 100wt%, wherein the given percentages relate to the total weight of the composition. Optionally the composition can comprise 0,01 to 2,0 wt% antioxidants and/or 0,1 to 3,0 wt% additional cosmetically active substances.

The hydrophilic complex which is used in the composition according to the invention is composed of 1,5 to 3,0 wt% of Rosa centifolia flower extract, 72,0 - 73,5 wt% water, more than 23 wt% glycerine, less than 1 wt% potassium sorbate and less than 1 wt% citric acid. This complex does provide a significant soothing effect as shown in *in vitro* tests on H₂O₂ stressed normal human epidermal keratinocytes (compare test description in the examples). This significant soothing effect is caused by the Rosa centifolia flower extract which was obtained by cryoextraction of Rosa centifolia petals coming preferably from Grasse roses. The first step of the extraction method consists in freezing the fresh flowers, preferably in the place of harvest, at -10 to - 40°C, preferably at -28°C. Then the flowers are subjected to cryopulverization under nitrogen at about - 196°C and subsequent extraction by maceration of the obtained cryopulverized powder in water.

The Rosa centifolia flower water which is used in the composition of the invention is prepared by steam distillation of Rosa centifolia petals, preferably of Grasse roses. The distillate contains more than 98% of Rosa centifolia flower water, <1% citric acid, 0,55% water, 0,3% sodium benzoate and 0,15% potassium sorbate.

The composition of the invention comprises anti-irritants. The anti-irritants are contained in the composition preferably from 0,1 to 0,8 wt%. According to the invention preferred used anti-irritants are Bisabolol (and) Farnesol (Trade name: Dragosantol) and/or Panthenol.

According to the invention the composition can optionally comprise known and in cosmetics widely used antioxidants. The antioxidants are contained in the composition in a range of 0,01 to 2,0 wt%, in a preferred range of 0,05 to 1,0 wt% and more preferred from 0,08 to 0,2 wt%. Suitable antioxidants are for instance vitamin C and its derivates, e.g. ascorbyl acetate, ascorbyl phosphate, ascorbyl palmitate. An especially preferred vitamin C derivate of the invention is Ascorbyl Glucoside (Trade name: Ascorbic Acid 2 Glucoside).

According to the invention the composition can comprise additional active substances in a range of 0,1 to 3,0 wt%. In a preferred embodiment of the invention these additional cosmetically active substances are Calendula Oil (Pale, NA20385; Caprylic/Capric Triglyceride & Glycine Soja (Soybean) Oil & Calendula Officinalis Flower Extract) and/or Camelina Sativa Seed Oil (Trade name: Huile Cameline Raffinee C05002). Each of them can be contained in the composition from 0,1 to 3,0 wt%. In a preferred embodiment both of them are contained, preferably in a range of 0,5 to 2.0 wt%.

The composition of the invention comprises auxiliary substances which are selected from the group comprising one or more of emulsifiers, buffers, chelating agents, preservatives, UV filters, colorants, fragrances, emollients, moisturizing substances, gelling agents, skin conditioning agents, opacifiers, solvents as for instance ethyl alcohol, and mixtures thereof.

The cosmetic composition of the invention is formulated as O/W or W/O emulsion, preferably as O/W emulsion. The emulsifiers which can be used for this purpose are well known by the skilled person. The emulsifiers are included in the composition of the present invention in a range of 2,5 to 7,0 wt%. According to the invention especially preferred emulsifiers are PPG-1-PEG-9 Lauryl Glycol Ether (Trade name: Eumulgin L), PEG-60 Hydrogenated Castor Oil (Trade name: Cremophor CO 60), Cetyl Alcohol and Glyceryl Stearate and PEG-75 Stearate and Ceteth-20 and Steareth-20 (Trade name: Emulium Delta), Cetearyl Alcohol (Trade name: Nafol 1618), Hydroxyethyl Acrylate/Sodium Acryloyldimethyl Taurate Copolymer and water and Squalane and Polysorbate 60 and Sorbitan Isostearate (Trade name: Simulgel NS).

According to the invention emollients can be added to soften and smoothen the skin. If emollients are used, they are included into the composition in the range of 1,0 to 13 wt%. Suitable emollients are for instance silicones, such as dimethicone, Polysilicone-11, siloxanes, such as Cyclopentasiloxane, Cyclohexasiloxane; Butyrospermum Parkii (Shea) Butter; Caprylic/Capric Triglyceride and Hydrogenated Polyisobutene. Further suitable emollients are for example Cetearyl Isononanoate, Octyldodecanol, Isopropyl Palmitate, Isopropyl Myristate, Mineral Oils, Isohexadacane, Diisopropyl Sebacate, C12-15 Alkyl Benzoate, Propylheptyl Caprylate, Pentaerythrityl tetraisostearate, Cetearyl Isononanoate, Isononyl Isononanoate, Ethyl Hexyl Hydroxystearate, Phenoxyethyl Caprylate, Isoamyl Cocoate.

In an embodiment of the invention the composition contains moisturizing substances in a range of 0.5 to 10,0 wt%. Preferred moisturizers which may be used are glycerine, propylene glycol, butylene glycol and Sorbitol/Water/Algae Extract/Potassium Sorbate/Sodium Benzoate.

Usual UV- filters / light absorbers can be added to the composition of the invention to protect the product from UVA and UVB radiation. Suitable oil and water soluble UVA and UVB filters are for instance benzophenones and their derivatives, such as benzophenone-4 or benzophenone-3.

In an embodiment of the invention the composition can comprise colorants. As a colorant cosmetically acceptable pigments, e.g. multilayer interference pigments, organic or inorganic dyes or mixtures thereof may be used. As a preferred example of a multilayer interference pigment Mica/Silica/Titanium dioxide is used.

The composition of the invention may comprise opacifying agents, if necessary. Preferred opacifiers are Styrene/Acrylate Copolymers, e.g. Sodium Styrene/Acrylates Copolymer (Trade name: Acusol OP 301). The opacifiers may be included from 0,1 to 2,5 wt%, preferably from 1,5 to 2,5%.

In a preferred embodiment of the invention the composition comprises fragrances in a range of 0,9 to 1,5 wt% to improve the odor of the cosmetic product. Preferably natural fragrances, such as plant or fruit extracts are used.

In one preferred embodiment of the invention the composition is a lotion which comprises 0,1 to 2,0 wt%, preferably 0,3 to 0,8 wt%, of the hydrophilic complex with the Rosa centifolia flower extract, 1,0 to 10,0 wt%, preferably 2,0 to 8,0 wt% of the Rosa centifolia flower water and 0,1 to 0,4 wt% Panthenol. The composition comprises 81,0 to 85,0 wt% of water and the remainder up to 100% auxiliary substances. It has been found by the inventors of the present invention that besides the soothing and hydrating effect the lotion leaves the skin glowing and purified.

In another preferred embodiment of the invention the composition is a cream and comprises as active substances 0,1 to 3,0 wt% of the hydrophilic complex with the Rosa centifolia flower extract, 1,0 to 10,0 wt%, preferably 1,0 to 2,0 wt%, of Rosa centifolia flower water , 0,1 to 1,0 wt%, preferably 0,3 to 0,8 wt%, of Bisabolol (and) Farnesol, 0,01 to 2,0 wt%, preferably 0,08 to 0,2 wt%, of Ascorbyl Glucoside, 55,0 to 60,0 wt% water and up to 100 wt% auxiliary substances. In addition to soothing activity and hydrating effect the cream also shows a very good antioxidant effect. The Radical Protection Factor (RPF) which determines the activity in respect of the bonding of free radicals by an antioxidant was measured as described in WO 99/66881. The RPF of the cream was found to be 150 · 10¹⁴ radicals/mg.

In a further preferred embodiment of the invention the composition is an emulsion and comprises as active substances 0,1 to 3,0 wt%, preferably 0,1 to 2,0 wt%, of the hydrophilic complex with the Rosa centifolia flower extract, 1,0 to 10,0 wt%, preferably 1,0 to 3,0 wt%, of Rosa centifolia flower water, 0,1 to 1,0 wt%, preferably 0,3 to 0,5 wt%, of Bisabolol (and) Farnesol, 0,1 to 2,0 wt% of Calendula Oil, 0,1 to 2,0 wt% Camelina Sativa Seed Oil and up to 100 wt% auxiliary substances. In addition to the soothing and hydrating effects the emulsion also shows a good protection against free radicals. The RPF of the emulsion was measured as explained above and found to be 60 · 10¹⁴ radicals/mg.

The emulsion of the invention has a melting gel-cream texture and is consistent and light at the same time. The emulsion is a comfortable, non greasy skin finish and provides a protected skin

The composition of the present invention has excellent soothing activity and significant and long lasting moisturizing effect on skin. Therefore, it is another object of the present application to provide a composition for use in treating skin areas subject to irritation linked to environmental conditions which comprises 0,1 to 3,0 wt% of a hydrophilic complex containing 1,5 to 3,0 wt% of Rosa centifolia flower extract obtainable by cryoextraction of Rosa centifolia flowers, 0,1 to 10,0 wt% of Rosa centifolia flower water, 0,1 to 1,0 wt% anti-irritants, optionally 0,01 to 2,0 wt% antioxidants, and also optionally 0,1 to 3,0 wt% additional cosmetically active substances, and water and cosmetically acceptable auxiliary substances up to 100wt%, wherein the given percentages relate to the total weight of the composition.

The compositions of the present invention are prepared in a manner as well known for O/W or W/O emulsions in cosmetic industries. Details of the preparation are given in the examples.

The following examples are offered to illustrate the cosmetic compositions of the present invention and their preparation. They are not intended to be limiting in any respect.

### Example 1: Lotion

| | | Batch 1 | Batch 2 | Batch 3 |
|---|---|---|---|---|
| | | % | % | % |
| **MAIN PHASE** | WATER | q.s. ad 100 | q.s. ad 100 | q.s. ad 100 |
| | TRISODIUM EDTA | 0,07 | 0,07 | 0,07 |
| | COLORANTS (0,1% IN WATER) FD & C RED N°4 AND FD & C YELLOW N°5 | 0,05 | 0,05 | 0,05 |
| | GLYCERIN | 5,00 | 4,00 | 3,00 |
| | ROSA CENTIFOLIA (GRASSE) FLOWER WATER | 2,00 | 4,50 | 7,50 |
| | ROSA CENTIFOLIA (GRASSE) HYDRO COMPLEX | 0,30 | 0,70 | 0,50 |
| | PANTHENOL | 0,30 | 0,30 | 0,30 |
| | ALGAE EXTRACT (HYDRO) | 1,00 | 1,00 | 1,00 |
| | PRESERVATIVE | 0,70 | 0,70 | 0,70 |
| | DISODIUM PHOSPHATE | 0,10 | 0,10 | 0,10 |
| | BENZOPHENONE-4 | 0,17 | 0,17 | 0,17 |
| | | | | |
| **PREMIX** | FRAGRANCE | 1,10 | 1,00 | 0,90 |
| | PPG-1-PEG-9 LAURYL GLYCOL ETHER | 2,00 | 1,95 | 1,85 |
| | PEG-60 HYDROGENATED CASTOR OIL | 1,20 | 1,10 | 1,00 |
| **PH ADJUSTER** | CITRIC ACID | q.s. ad 100 | q.s. ad 100 | q.s. ad 100 |

The composition of Example 1 is prepared as follows:
Water is added in the main vessel. The ingredients of the main phase are added separately under slowly stirring to the water. The stirring rate is preferably from 50 to 150 rpm and a paddle stirrer is preferably used. The premix is prepared under stirring and heating up to maximum 40°C about 5 to 10 minutes until homogeneity is achieved. The premix is added to the main phase and the pH is adjusted to skin neutral pH.

### Example 2: Cream

| | | Batch 1 | Batch 2 | Batch 3 |
|---|---|---|---|---|
| | | % | % | % |
| **PHASE A** | | | | |
| | CETYL ALCOHOL & GLYCERYL | | | |
| | STEARATE & PEG-75 STEARATE & | 6,35 | 6,35 | 6,35 |
| | CETETH-20 & CETEARYL ALCOHOL | | | |
| | CAPRYLIC/CAPRIC TRIGLYCERIDE | 5,50 | 5,50 | 2,50 |
| | BUTRYROSPERMUM PARKII (SHEA) BUTTER | 5,50 | 5,50 | 5,50 |
| | BHT | 0,04 | 0,04 | 0,04 |
| | HYDROGENATED POLYISOBUTENE | 4,00 | 3,50 | 4,00 |
| **PHASE B** | WATER | q.s. ad 100 | q.s. ad 100 | q.s. ad 100 |
| | GLYCERIN | 5,00 | 3,00 | 5,50 |
| | PROPYLENE GLYCOL | 2,50 | 2,00 | 2,50 |
| | TRISODIUM EDTA | 0,10 | 0,10 | 0,10 |
| | ASCORBYL GLUCOSIDE | 0,08 | 0,13 | 0,20 |
| | MICA & SILICA & TITANIUM DIOXIDE | 0,17 | 0,17 | 0,17 |
| | XANTHAN GUM | 0,25 | 0,25 | 0,25 |
| | AMMONIUM | | | |
| | ACRYLOYLDIMETHYLTAURATE/VP | 0,70 | 0,60 | 0,50 |
| | COPOLYMER | | | |
| **PHASE C** | TRIETHANOLAMINE | 0,025 | 0,025 | 0,025 |
| | CYCLOPENTASILOXANE & | 4,00 | 4,00 | 4,00 |
| | POLYSILICONE-11 | | | |
| | PTFE | 0,90 | 0,90 | 0,90 |
| | POLYMETHYL METHACRYLATE | 0,50 | 0,50 | 0,50 |
| **PHASE D** | ROSA CENTIFOLIA (GRASSE) HYDRO COMPLEX | 0,20 | 1,90 | 3,00 |
| | ROSA CENTIFOLIA (GRASSE) FLOWER WATER | 1,50 | 2,00 | 1,50 |
| | BISABOLOL & FARNESOL | 0,30 | 0,50 | 0,70 |
| | PRESERVATIVE | 1,00 | 1,00 | 1,00 |
| | FRAGRANCE | 1,50 | 1,35 | 1,20 |

The composition of Example 2 is prepared as follows:
Water of phase B is added in the main vessel. The ingredients of phase B are added separately to the water under slowly stirring until homogeneity is achieved. Phase A is separately prepared in a second vessel. Both phases are heated up to 80°C.
Phases A and B are mixed together in the main vessel under stirring until homogeneity is achieved. After cooling to 50°C the ingredients of phase C are separately added under stirring. The cream is cooled to 35°C and the ingredients of phase D are added under stirring until homogeneity.

### Example 3: Emulsion

| | | **Batch 1** | **Batch 2** | **Batch 3** |
|---|---|---|---|---|
| | | % | % | % |
| **PHASE A** | WATER | q.s. ad 100 | q.s. ad 100 | q.s. ad 100 |
| | GLYCERIN | 4,00 | 4,00 | 4,00 |
| | BUTYLENE GLYCOL | 1,50 | 1,50 | 1,50 |
| | MICA & SILICA & TITANIUM DIOXIDE | 0,15 | 0,15 | 0,15 |
| | ASCORBYL GLUCOSIDE | 0,13 | 0,13 | 0,13 |
| | HYDROXYETHYL ACRYLATE / SODIUM ACROYLDIMETHYL TAURATE COPOLYMER & WATER & SQUALANE & POLYSORBATE 60 & SORBITAN ISOSTEARATE | 2,80 | 2,80 | 2,80 |
| | CYCLOPENTASILOXANE & CYCLOHEXASILOXANE | 2,00 | 2,00 | 2,00 |
| | CYCLOPENTASILOXANE & DIMETHICONOL | 2,00 | 2,00 | 2,00 |
| | DIMETHICONE | 2,00 | 2,00 | 2,00 |
| **PHASE B** | CAMELINA SATIVA SEED OIL | 0,50 | 2,00 | 1,00 |
| | Calendula Oil | 0,50 | 2,00 | 1,00 |
| | ROSA CENTIFOLIA (GRASSE) HYDRO COMPLEX | 1,00 | 1,90 | 0,50 |
| | ROSA CENTIFOLIA (GRASSE) FLOWER WATER | 1,00 | 2,00 | 3,00 |
| | BISABOLOL & FARNESOL | 0,30 | 0,40 | 0,50 |
| | | | | |
| **PHASE C** | PRESERVATIVE | 0,70 | 0,70 | 0,70 |
| | FRAGRANCE | 1,00 | 1,10 | 1,20 |
| | ETHYLALCOHOL | 1,70 | 1,70 | 1,70 |
| | | | | |
| **PREMIX** | WATER | 5,00 | 5,00 | 5,00 |
| | WATER & STYRENE / ACRYLATES COPOLYMER SODIUM LAURYL SULFATE | 1,00 | 1,00 | 1,00 |
| **PHASE D** | TRIETHANOLAMINE | q.s. ad 100 | q.s. ad 100 | q.s. ad 100 |

The composition of Example 3 is prepared as follows:
Water of phase A is added to the main vessel and heated to 50°C. The ingredients of phase A are added separately to the water under slowly stirring until homogeneity.
Under stirring phase A is cooled below 40°C. The ingredients of phase B are added separately to phase A. After cooling below 30°C phase C is added separately. In a separate vessel the premix is prepared and added to the emulsion under continuous slowly stirring. At the end the pH is adjusted to skin neutral pH with Triethanolamine.

### Example 4: Determination of soothing activity of Rosa centifolia hydrophilic complex and Rosa centifolia flower water

Normal human epidermal keratinocytes were treated with
a) 0,5% of Rosa centifolia hydrophilic complex diluted in cell culture medium (DMEM) or
b) 0,5% of Rosa centifolia hydrophilic complex and 5% Rosa centifolia flower water diluted in cell culture medium (DMEM) for 2 hours.

Inflammation (stress) was then induced by H2O2 solution for 10 min and cells were incubated for additional 24 hours with a) or b).

In the same conditions non treated cells were stressed with H2O2 solution. Extra-cellular release of cytokines (IL1a, IL6, IL8) was quantified with Fluorokine™ MAP cytokine multiplex kit purchased from R&D Systems.

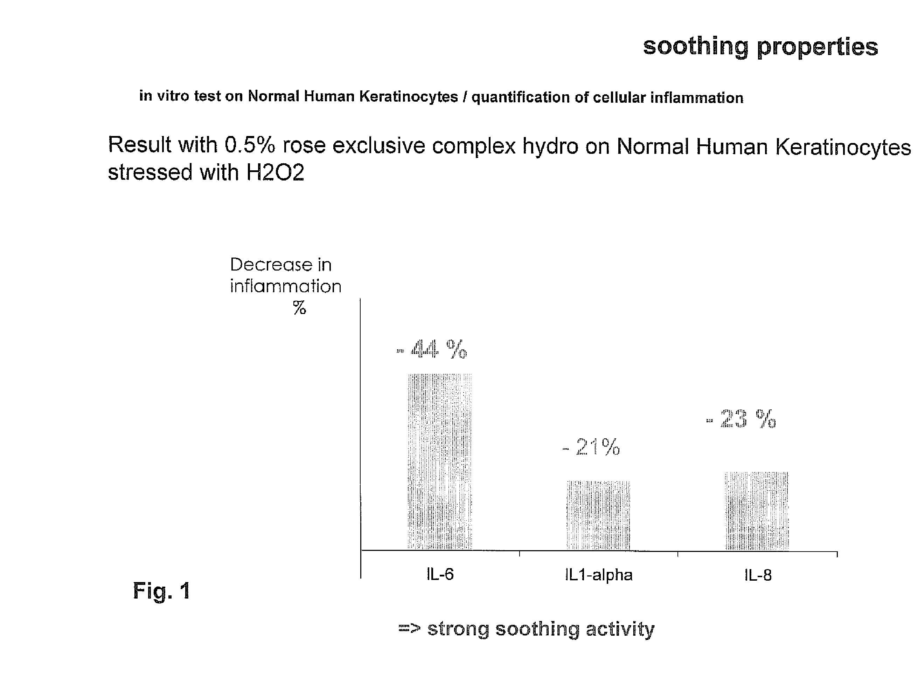

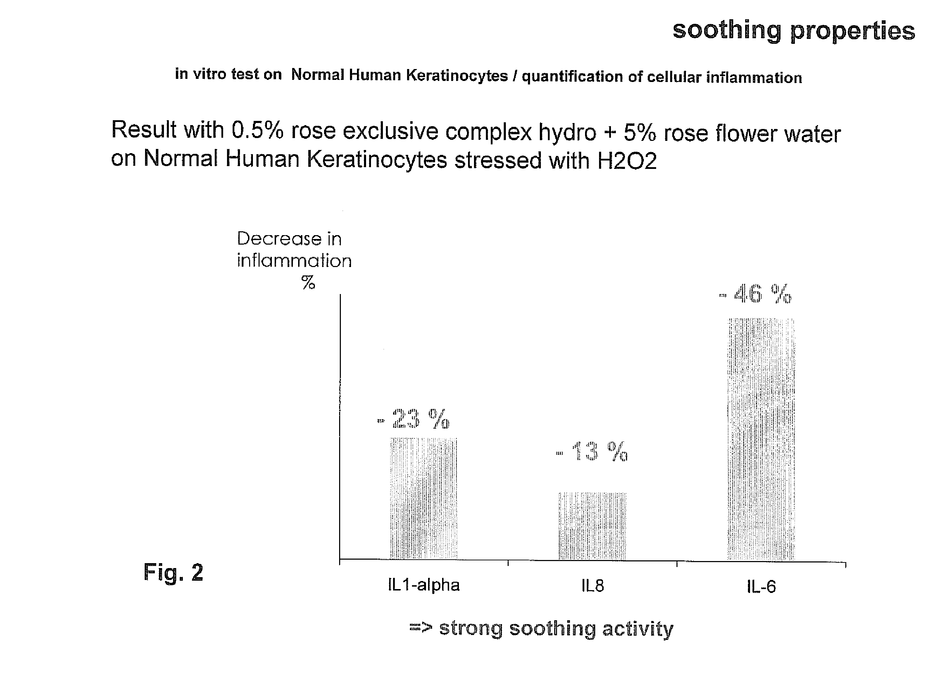

The results are given in Figures 1 and 2.
**Fig.1** shows the soothing effects obtained with 0,5% of the Rosa centifolia complex. The release of IL1α, IL8 and IL6 is reduced for 21%, 23% and 44%, respectively.
**Fig. 2** evidences that the release of IL1α, IL8 and IL6 is reduced for 23%, 13% and 46%, respectively, by 0,5% of Rosa centifolia complex together with 5% Rosa centifolia flower water.

### Example 5: Determination of moisturizing effect (skin hydration) of the compositions of Examples 1, 2 and 3

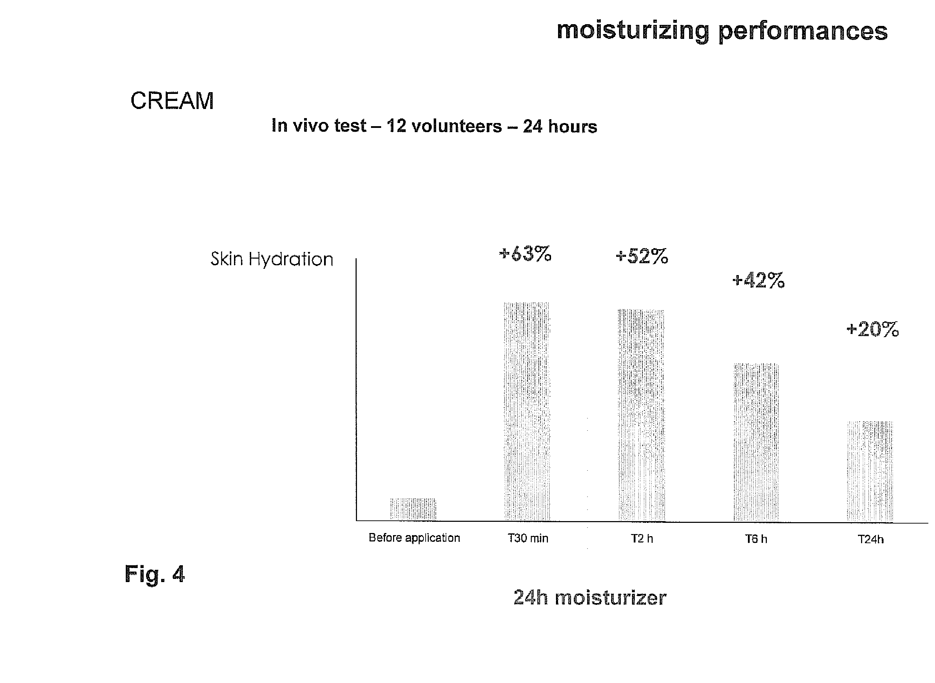

Skin hydration of the epidermal outermost layers of the arms of 12 women was assessed before application and after application of the compositions of Examples 1, 2 and 3 during 24 hours using the Corneometer™ (Courage & Khazaka Electronics). This technique is widely used to investigate skin water content and to quantify the effect of cosmetics or dermatological products. Due to the high dielectric constant of water, the electrical characteristics of the *stratum corneum* depend on its water content. The values of electrical capacitance are given in arbitrary units (A.U.) settled by the instrument on a scale from 0 A.U. to about 130 A.U. These values show evidence of the hydration degree of the outermost layers of the epidermis at a given time. The results are given in Figures 4, 5 and 6.

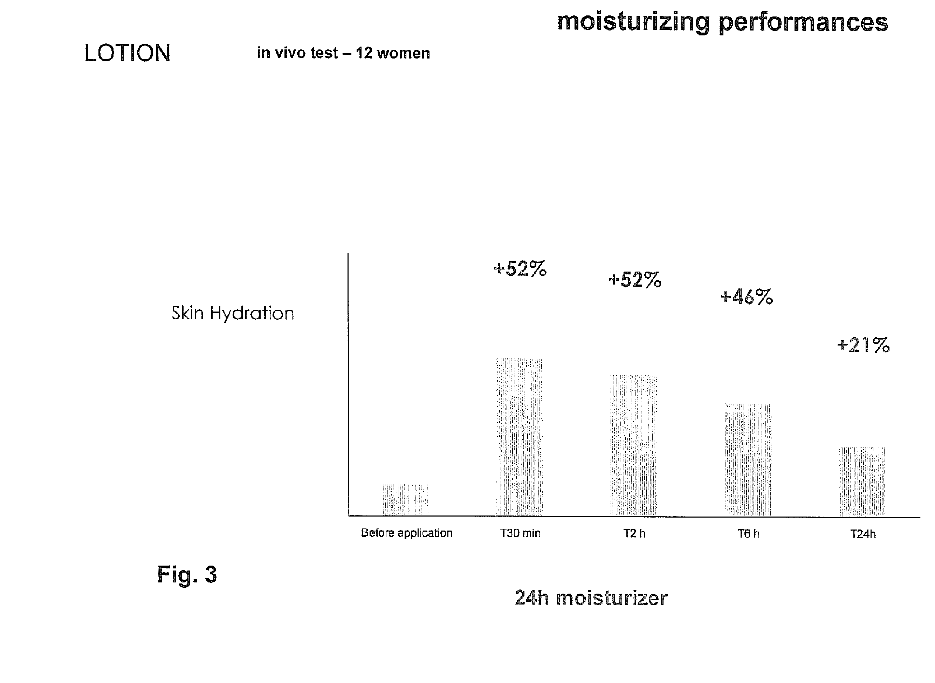

**Figure 3** shows that the skin moisture increases after application of the lotion of Example 1 for 52% after 30 minutes and is after 24 hours still 21 % higher than before application, so that the lotion of the invention is a 24 hours moisturizer.
**Figure 4** exhibits the moisturizing performances of the cream of Example 2. The cream also shows a significant and long-lasting hydrating effect. After 24 hours the skin moisture is still 20% higher than before application of the cream.

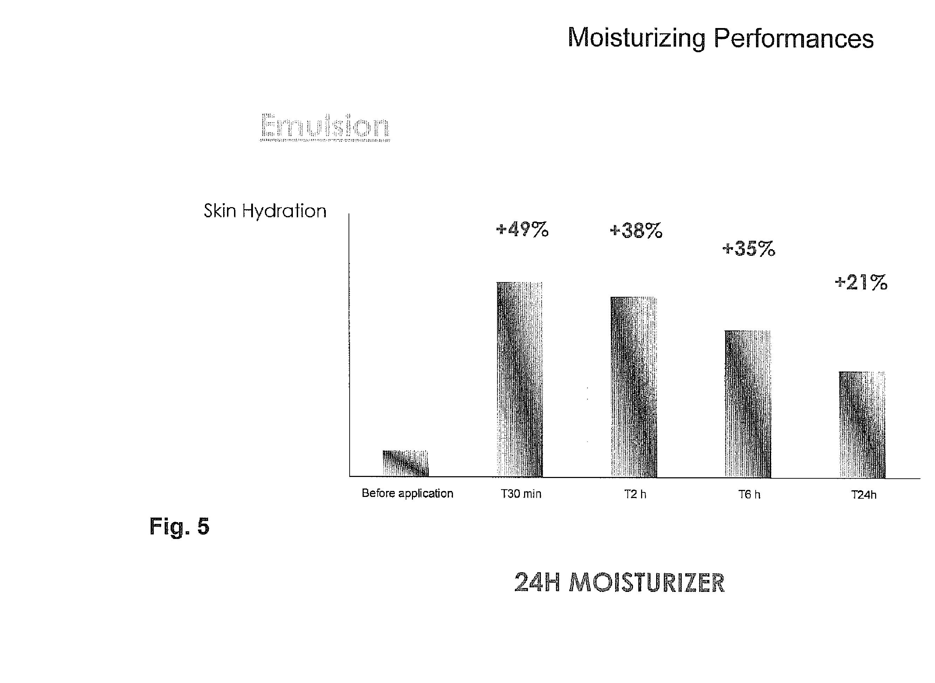

**Figure 5** shows the moisturizing performances of the emulsion of Example 3. The skin moisture increases after application of the emulsion for 49% after 30 minutes and is after 24 hours still 21 % higher than on non treated areas at the same times of measurements.

## Claims

1. A skin composition with soothing effect comprising
0,1 to 3,0 wt% of a hydrophilic complex containing 1,5 to 3,0 wt% of Rosa centifolia flower extract obtainable by cryoextraction of Rosa centifolia flowers,
0,1 to 10,0 wt% of Rosa centifolia flower water and 0,1 to 1,0 wt% anti-irritants, optionally 0,01 to 2,0 wt% antioxidants, and also optionally 0,1 to 3,0 wt% additional cosmetically active substances,
and water and cosmetically acceptable auxiliary substances up to 100wt%, wherein the given percentages relate to the total weight of the composition.

2. The composition according to claim 1,
wherein the composition comprises 0,1 to 0,8 wt% anti-irritants.

3. The composition according to claim 1 or 2,
wherein the anti-irritant is Bisabolol (and) Farnesol and/or panthenol.

4. The composition according to any one of claims 1 to 3,
wherein the composition comprises as additional cosmetically active substances Calendula Oil and/or Camelina Sativa Seed Oil.

5. The composition of any one of claims 1 to 4,
wherein the composition comprises 0,5 to 2,0 wt% Calendula Oil and/or 0,5 to 2,0 wt% Camelina Sativa Seed Oil.

6. The composition according to any one of claims 1 to 5,
wherein the composition comprises 0,05 to 1,0 wt% antioxidants, preferably 0,08 to 0,2 wt%.

7. The composition according to any one of claims 1 to 6,
wherein the composition comprises as antioxidant Ascorbyl Glucoside.

8. The composition according to any one of claims 1 to 7,
wherein the composition comprises 0,3 to 2,0 wt% of the hydrophilic complex.

9. The composition according to any one of claims 1 to 8,
wherein the composition comprises 1,0 to 5,0 wt% of Rosa centifolia flower water.

10. The composition according to any one of claims 1 to 9,
wherein the composition comprises cosmetically acceptable auxiliary substances which are selected from the group comprising emulsifiers, buffers, preservatives, UV filters, colorants, fragrances, silicone emollients, moisturizing substances, gelling agents, skin conditioning agents, opacifiers, and mixtures thereof.

11. The composition according to any one of claims 1 to 10,
wherein the composition is a lotion comprising 0,1 to 2,0 wt%, preferably 0,3 to 0,8 wt%, of the hydrophilic complex with the Rosa centifolia flower extract, 1,0 to 10,0 wt% of the Rosa centifolia flower water, 0,1 to 0,4 wt% Panthenol, 81,0 to 85,0 wt% water and up to 100% auxiliary substances.

12. The composition according to any one of claims 1 to 10,
wherein the composition is a cream comprising 0,1 to 3,0 wt% of the hydrophilic complex with the Rosa centifolia flower extract, 1,0 to 10,0 wt%, preferably 1,0 to 2,0 wt%, of the Rosa centifolia flower water, 0,1 to 1,0 wt% of Bisabolol (and) Farnesol, 0,01 to 2,0 wt%, preferably 0,08 to 0,2 wt%, of Ascorbyl Glucoside, 55 to 60 wt% water and up to 100 wt% auxiliary substances.

13. The composition according to any one of claims 1 to 10,
wherein the composition is an emulsion comprising 0,1 to 3,0 wt% of the hydrophilic complex with the Rosa centifolia flower extract, 1,0 to 10,0 wt%, preferably 1,0 to 3,0 wt%, of the Rosa centifolia flower water, 0,1 to 1,0 wt% of Bisabolol (and) Farnesol, 0,1 to 2,0 wt% of Calendula Oil, 0,1 to 2,0 wt% of Camelina Sativa Seed Oil and up to 100 wt% auxiliary substances.

14. A composition for use in soothing the skin by applying it on skin areas irritated by environmental conditions, wherein the composition comprises
0,1 to 3,0 wt% of a hydrophilic complex containing 1,5 to 3,0 wt% of Rosa centifolia flower extract obtainable by cryoextraction of Rosa centifolia flowers, 0,1 to 10,0 wt% of Rosa centifolia flower water and 0,1 to 1,0 wt% anti-irritants, optionally 0,01 to 2,0 wt% antioxidants, and also optionally 0,1 to 3,0 wt% additional cosmetically active substances,
and water and cosmetically acceptable auxiliary substances up to 100wt%, wherein the given percentages relate to the total weight of the composition.

15. A composition according to claim 11, wherein the composition is applied 2 to 3 times daily.
